# EUROPEAN PATENT APPLICATION

(11) **EP 1 762 570 A1**
(43) Date of publication of application: **14.03.2007**
(21) Application number: 06118176.4
(22) Date of filing: 31.07.2006
(51) Int. Cl.: C07D 501/00

(54) **Process for producing the cefepime dihydrochloride monohydrate**

(30) Priority: 13.09.2005 IT MI20051684
(71) Applicant: Harvest Lodge Limited, London, WC2A 3LJ (GB)
(72) Inventor: Zenoni, Maurizio, 20067, Paullo (MI) (IT); Filippi, Mauro, 20060, Bettolino di Mediglia (MI) (IT)
(74) Representative: Frignoli, Luigi

(57) **Abstract**

The cefepime sulphate is transformed and isolated as the dihydrochloride, by neutralizing the sulphuric acid with sodium hydroxide, filtering off the precipitated sodium sulphate and reacidifying with hydrochloric acid.

## Description

The amino acids to which the present invention relates are antibiotics, in particular cephalosporins, and more particularly a cephalosporin known as cefepime.

Cefepime is a cephalosporin with a broad spectrum of action; it is claimed in US 4406899 and is administered by injection as the dihydrochloride monohydrate claimed in US 4910301. Preparation of the dihydrochloride monohydrate is described in certain patents: US 4910301, US 4994451, US 5244891, US 5594129. Preparation takes place essentially by treating the sulphate obtained in the synthesis with an ion exchange resin to release the pure zwitterion from which the desired dihydrochloride salt is prepared. According to the process described in example XI of US patent 4910301, a yield equal to 88.6% of the theoretical is obtained, but the operations are rather lengthy and complex. Schematically, the aqueous solution obtained after treatment with ion exchange resin and containing the zwitterion is acidified with 6N HCl after extracting with solvent, evaporating the residual solvent, decolorizing and filtering. Acetone is added dropwise to the aqueous acid solution thus obtained until precipitation of the dihydrochloride is complete, while the resin is regenerated separately.

The object of the present patent application is to describe an industrial process that is extremely simple, fast and with a higher yield than even the already satisfactory one of the aforestated US 4910301.

In this respect, it has surprisingly been found that treating with resin to release the zwitterion is unnecessary as it lengthens the procedure and involves reactors, filters etc. including those for resin regeneration, however the cefepime sulphate derived from the synthesis can be transformed directly into sterile cefepime dihydrochloride monohydrate without using organic solvents except the crystallization solvent. The aforesaid cefepime sulphate is in fact fed into water and transformed into the corresponding zwitterion by adding an aqueous sodium hydroxide solution; the thus obtained solution is decolorized, diluted with acetone and filtered to remove precipitated sodium sulphate. A solution is therefore obtained from which the dihydrochloride is easily crystallized by the addition of 6N HCl.

The process outlined above will now be described in detail in the following example.

### EXAMPLE 1

300 g of cefepime sulphate with a purity of 80.65% are fed into 1300 ml of deionised water which has been cooled and maintained at 0°/+5°C, an aqueous sodium hydroxide solution then being added dropwise until the pH is 7.7.

The aqueous solution is diluted with 6 l of acetone. The sodium sulphate precipitate is filtered off and washed with a solution of acetone and deionised water. The solution obtained is sterilely filtered and washed with sterile water.

The solution is diluted with 1 l of acetone and 6N HCl is added dropwise at ambient temperature to pH 0.5. 10 l of acetone are then added in a thin stream at ambient temperature over 30 minutes, and agitation is continued to complete the crystallization at ambient temperature.

The product is filtered off and washed with acetone, then dried under vacuum at 40°C to a K.F. between 3.0% and 4.5%.

Yield: 260 g of cefepime dihydrochloride monohydrate with purity of 84.63%, equal to a 91 % yield on the theoretical.

By operating as described in example 1, similar results can be obtained by using cefepime sulphate with a purity of between 75% and 85%, and by adding an aqueous sodium hydroxide solution until the pH is between 7 and 8.

## Claims

1. A process for producing sterile cefepime dihydrochloride monohydrate, **characterised by** dissolving synthesis-produced cefepime sulphate in water at pH 7.0-8.0, at a temperature between 0° and +5°C, by adding an aqueous sodium hydroxide solution at a concentration of between 15% and 30%, then diluting the solution with a water-miscible organic solvent to achieve precipitation of the sodium sulphate formed, filtering off the precipitate, then filtering the solution under sterile conditions and acidifying said solution with 6N HCl to pH 0.4-0.6, then diluting with the same already used organic solvent until complete precipitation of the dihydrochloride monohydrate, which is then filtered off, washed with acetone and dried under vacuum to a K.F. of between 3.0% and 4.5%.

2. A process as claimed in claim 1, **characterised in that** said acidification of the solution with 6N HCl is undertaken until the pH is 0.5.

3. A process as claimed in claims 1 and 2, **characterised in that** the cefepime sulphate has a purity of between 75% and 85%.

4. A process as claimed in claims 1 to 3, **characterised in that** said organic solvent is acetone.
